# EUROPEAN PATENT SPECIFICATION

(11) **EP 1 578 772 B1**
(45) Date of publication and mention of the grant of the patent: **12.05.2010**
(21) Application number: 03713721.3
(22) Date of filing: 26.02.2003
(51) Int. Cl.: A61K 38/00, A61K 45/00, A61K 47/00, G01N 33/53, C12N 5/00, C12N 5/02, C07K 1/00, C07K 14/00, C07K 16/00, G01N 33/50, C07K 14/52

(54) **CYTOKINES AND CYTOKINE RECEPTORS WITH REDUCED IMMUNOGENICITY**
CYTOKINE UND CYTOKINREZEPTOREN MIT REDUZIERTER IMMUNOGENITÄT
CYTOKINES ET RECEPTEURS DE CYTOKINES PRESENTANT UNE IMMUNOGENECITE REDUITE

(30) Priority: 01.05.2002 US 376743 P
(43) Date of publication of application: 28.09.2005
(73) Proprietor: Danisco US Inc., Palo Alto, CA 94304 (US)
(72) Inventor: HARDING, Fiona, A., Santa Clara, CA 95050 (US); POWER, Scott, D., San Bruno, CA 94066 (US)
(74) Representative: Duxbury, Stephen
(86) International application number: PCT/US2003/005917
(87) International publication number: WO 2003/104263

(56) References cited:
- WO-A-83/02461
- WO-A2-99/53038
- US-A- 4 289 689
- SOTIRIADOU R ET AL: "Peptide HER2(776-788) represents a naturally processed broad MHC class II-restricted T cell epitope." BRITISH JOURNAL OF CANCER. 16 NOV 2001, vol. 85, no. 10, 16 November 2001 (2001-11-16), pages 1527-1534, XP002400860 ISSN: 0007-0920
- STICKLER M M ET AL: "CD4+ T-CELL EPITOPE DETERMINATION USING UNEXPOSED HUMAN DONOR PERIPHERAL BLOOD MONONUCLEAR CELLS" JOURNAL OF IMMUNOTHERAPY, LIPPINCOTT WILLIAMS & WILKINS, HAGERSTOWN, MD, US, vol. 23, no. 6, November 2000 (2000-11), pages 654-660, XP008040520 ISSN: 1524-9557
- PEIPER M ET AL: "Generation of peptide-specific cytotoxic T lymphocytes using allogeneic dendritic cells capable of lysing human pancreatic cancer cells." SURGERY. AUG 1997, vol. 122, no. 2, August 1997 (1997-08), pages 235-241 ; dis, XP002400858 ISSN: 0039-6060
- RONGCUN Y ET AL: "Interferon gamma impairs the ability of monocyte-derived dendritic cells to present tumour-specific and allo-specific antigens and reduces their expression of CD1A, CD80 AND CD4." CYTOKINE. OCT 1998, vol. 10, no. 10, October 1998 (1998-10), pages 747-755, XP002400859 ISSN: 1043-4666
- MUCHA J.M. ET AL: 'Enhanced immunogenicity of a functional enzyme by T cell epitope modification' BMC IMMUNOL vol. 3, no. 2, 25 January 2002, pages 1 - 12, XP001206375
- TAKAMIZAWA M. ET AL: 'Dendritic Cells That Process and Present Nominal Antigens to Naive T Lymphocytes Are Derived from CD2+ Precursors' J. IMMUNOL. vol. 158, no. 5, March 1997, pages 2134 - 2142, XP002994301

## Description

### FIELD OF THE INVENTION

The present disclosure provides means and compositions suitable for reducing the immunogenicity of cytokines and cytokines receptors such as interferon-β.

### BACKGROUND OF THE INVENTION

Treatment with cytokines has become increasingly popular for various conditions. For example, thrombopoietin (TPO) has been considered for use as a protein therapeutic in the treatment of platelet loss due to genetic abnormalities or myeloablative cancer therapies. Unfortunately, TPO has been shown to induce the production of neutralizing antibodies that result in persistent autoimmune thrombocy topenia (Li et al., Blood 98:3241-8 [2001]). Erythropoietin (EPO) has found use in the treatment of anemia of end-stage renal disease and zidovudine anemia in AIDS patients. However, serious complications have been associated with the administration of EPO, including the development of aplastic anemia and other autoimmune-related problems (Casadev all et al., New Eng. J. Med., 346:469-475 [2002]).
The cytokine receptor, soluble tumor necrosis factor receptor 1 (TNF-R1) has been considered for use as a therapeutic for anti-inflammatory applications. However, as with TPO, TNF-R1 has also been shown to induce the production of neutralizing antibodies (Corcoran et al., Eur Cytokine Netw 9:255-262 [1998]).
Yet another cytokine, interferon β (IFN-β), is emerging as a potentially effective form of therapy for various immune-mediated conditions, cancer and other diseases. Indeed, IFN-β has been investigated for use in treatment of rheumatoid arthritis, chronic hepatitis C infection, leptomeningeal metastasis, colorectal liver metastasis, malignant melanoma, allergic encephalomyelitis, brain stem gliomas, chronic inflammatory polyneuritis and demyelinating diseases. Although it has found use in many settings, IFN-β therapy has found greatest use in treatment of diseases such as multiple sclerosis.
Multiple sclerosis (MS) is an aggressive, inflammatory-mediated destructive demyelinating disease of the central nervous system, believed to be mediated by an autoimmune process. The disease is associated with demyelination, destruction of oligodendrocytes and axonal injury (Trapp et al. N. Engl. J. Med., 338:278-285 [1998]). The consequences include the production of various cytokines specific for individual disease stages (Amason, Int'l. Multiple Sclerosis J., 4:40-42 [1997]; and Link, Multiple Sclerosis, 4:12-15 [1998]), characterized by a particular Th1 profile during relapse (Correale et al. J. Immunol., 154:2959-2968 [1995]; and Hermans et al., Ann. Neurol., 42:18-27 [1997]) and a characteristic pattern of proinflammatory cytokines such as interleukin-2 (IL-2), interferon-γ (IFN-y) and tumor necrosis factor-α (TNF-α). These cytokines are down-regulated during non-active disease. It is believed that the immunomodulatory properties of interferon-β (IFN- β) can slow the progression of the disease, perhaps by creating a cytokine environment more similar to the non-active state. In addition, during relapse, interferon-β production appears to be suppressed (Pachner Neurol., 49:647-650 [1997]; and Wandinger et al. J. Neurol. Neurosurg. Psych., 64:277-278 [1998]). Currently, there are three pharmaceutical versions of interferon-β that have been approved for clinical use; each interferon variant is known to possess unique immunological properties (See, Colby et al. J. Immunol., 133:3091-3095 [1984]) and unique therapeutic properties (Rolak, Neurol. Treat., 19:107118 [2001]).
Cytokines comprise a network of soluble cell factors, which acting through receptors, regulate the activation state of the immune system (Perini et al. Eur. Cytokine Network 12:56-91 [2001]). Autoantibodies to these soluble cell factors, including cytokines and cytokine receptors, are known but only occur rarely in normal individuals. In contrast, in patients with autoimmune and inflammatory diseases, high titres of autoantibodies directed against specific cytokines and/or their receptors are sometimes detected. Two types of antibodies are recognized, namely, binding antibodies (Bab) and neutralizing antibodies (Nab). The role of Babs is still debated, but is believed to involve regulating the bioavailability of a particular protein (*e.g*., a cytokine or receptor) and/or its turnover (Pachner, Neurology 49:647 [1997]). Neutralizing antibodies are directed toward the portion of the cytokine (or receptor) molecule required for target binding, ligation or function. In contrast to Babs, neutralizing antibodies may directly interfere with a protein's ability to exert its biological function.
The development of Nabs against TPO, TNF-R1, and IFNs has been well-documented (See, Antonelli, Antiviral Res. 24:235-244 [1994], for a discussion of the development of Nabs during IFN-β treatment of cancer and viral hepatitis). The appearance of high titres of Nabs is associated with a reduction in interferon-mediated adverse events (chills, fever, myalgia) as well as a recurrence of disease. In these cases the effect of Nabs can be followed through surrogate markers such as the appearance of enzyme markers for disease (transaminase and hepatitis) and antigen markers for cancers (Rice, Arch. Neurol., 58:1297-1298 [2001]).
Nabs directed against IFN-β are known to form in a subset (relapsing remitting) multiple sclerosis patients (MS) undergoing interferon therapy. Both IFN-β1a and IFN-β1b have been shown to induce the formation of Nabs. In the case of INFβ1b, the appearance of high Nab titer in the observation period between 18 and 24 months was associated with a loss in clinical efficacy (IFNB MS Study Group et al. Neurol., 47:889-894 [1996]). Inf-β1a administration has been shown to also reveal significant differences between Nab positive and Nab negative patients in attack, disability and MRI outcome long term (Rice, Programs and Abs. Europ. Clin. Trials Meeting in MS, Toulouse, France (September 6-9, 2000); PRISMS study group, Lancet 352:1498-1504 [1998]). Therefore, it is contemplated that the appearance of Nabs to INF-βs during clinical administration can lead to treatment failures (See, Whitaker, Arch. Neurol., 58: 1295-1296 [2001]; Rice, Arch. Neurol., 58:1297-1298 [2001], Pachner, Arch. Neuro., 58: 1299-1300 [2001]; Li et al. Neurology 56:1505-1513 [2001]; and Whitaker, Arch. Neurol., 58:1295-1296 [2001]). Thus, means are needed to prevent the immunopathology associated with the administration of cytokines and/or cytokine receptors such as TPO, TNF-R1, and IFN-β to patients.
Peiper M. Et al.; Surgery 122; 1999; pp 235-242, Rongcun Y. et al.; Cytokine 10(10); 1998; pp 747-755, and Sotiriadou R. et al.; British Journal of Cancer 85(10); 2001; pp 1527-1534 disclose methods for determining a T-cell epitope of a cytokine receptor, namely human EGF receptor 2 (HER2).

### SUMMARY OF THE INVENTION

The present disclosure provides means and compositions suitable for reducing the immunogenicity of cytokines and cytokines receptors such as interferon-β. The cytokines and cytokine receptors are human cytokines and cytokine receptors. The scope of the invention is defined by the claims.

Disclosed are methods for determining a T-cell epitope of a protein, wherein the protein is selected from the group consisting of cytokines and cytokine receptors, comprising the steps of: (a) obtaining from a solution of dendritic cells and a solution of naïve CD4+ and/or CD8+ T-cells from a single human blood source; (b) differentiating the dendritic cells, in the solution of dendritic cells, to produce a solution of differentiated dendritic cells; (c) preparing a pepset of peptides from the protein; (d) combining the solution of differentiated dendritic cells and naive CD4+ and/or CD8+ T-cells with the pepset, wherein the pepset comprises the T-cell epitope; and (e) measuring the proliferation of the T-cells in step (d). The protein is selected from the group consisting of interferons, cytokine receptors, hematopoietic factors, interferon-beta, soluble tumor necrosis factor receptor 1, and thrombopoietin. The protein comprises an amino acid sequence selected from the group consisting of SEQ ID NO:1, SEQ ID NO:104, SEQ ID NO:106, and SEQ ID NO:108. The pepset comprises a peptide having the sequence selected from the group consisting of SEQ ID NO:2, SEQ ID NO:28, and SEQ ID NO:41. In some further disclosures, the pepset comprise a peptide having the sequence set forth in SEQ ID NO:105, while in other further disclosures, the pepset comprises a peptide having the sequence set forth in SEQ ID NO:107. In yet additional disclosures, the pepset comprises a peptide having the sequence selected from the group consisting of SEQ ID NO: 109 and SEQ ID NO:110.
In additional disclosure, the methods further comprise the step of modifying the protein to produce a variant protein, wherein the variant protein exhibits an altered immunogenic response as compared to the parent (*i.e*., originating or source) protein. In yet other disclosures, the variant comprises an epitope having the sequence of SEQ ID NO:41. In some preferred embodiments, the variant protein comprises at least one amino acid substitution at an amino acid residue selected from the group consisting of position 125 and position 129. In some disclosures of the variant proteins, the substitution at position 125 is selected from the group consisting of alanine, leucine, and lysine. In some alternative disclosures the variant protein comprises a substitution at position 129 selected from the group consisting of leucine, alanine, and valine. In further disclosures, variant protein comprises at least one amino acid substitution at an amino acid residue selected from the group consisting of position 2, position 4, position 5, position 8, position 11, position 17, position 118, position 124, and position 128. In still further disclosures, the variant protein comprises an epitope having the sequence of SEQ ID NO:105. In additional disclosures, the variant protein comprises at least one amino acid substitution at an amino acid residue selected from the group consisting of position 138, position 139, and 140. In some disclosures of the variant protein, the substitution at position 138 is a serine, while in other disclosures, the substitution at position 139 is an alanine, and in still further disclosures, the substitution at position 140 is an alanine. These substitutions of positions 138, 139, and 140 are present in any combination, including single substitutions (*i.e*., only one of these positions is altered), double substitutions (*i.e*., two of the positions are altered), and triple substitutions (*i.e*., all three of these positions are altered). Thus, it is not intended that the present disclosure be limited to any particular combination of substitutions or other changes to the amino acid sequence of the protein.
The present disclosure further provides amino acid sequences, including, but not limited to SEQ ID NO:2, SEQ ID NO:28, SEQ ID NO:41, SEQ ID NO:45, SEQ ID NO:105, SEQ ID NO:107, SEQ ID NO:109, and SEQ ID NO:110.
The present disclosure also provides methods for reducing the immunogenicity of a protein, wherein the protein is selected from the group consisting of cytokines and cytokine receptors, comprising the steps of: (a) identifying at least one T-cell epitope in the protein by (i) contacting an adherent monocyte-derived dendritic cell that has been differentiated by exposure to at least one cytokine *in vitro,* with at least one peptide comprising the T-cell epitope; and (ii) contacting the dendritic cell and said peptide with a naïve T-cell, wherein the naïve T-cell has been obtained from the same source as the adherent monocyte-derived dendritic cell, and whereby the T-cell proliferates in response to the peptide; and (b) modifying the protein to neutralize the T-cell epitope to produce a variant protein, such that the variant protein induces less than or substantially equal to the baseline proliferation of the naïve T-cells. The T-cell epitope is modified by substituting a portion of the amino acid sequence of the T-cell epitope with an analogous sequence from a homolog of the protein. Alternatively, the T-cell epitope is modified by substituting the amino acid sequence of the T-cell epitope with a sequence which substantially mimics the major tertiary structure attributes of the T-cell epitope. Preferably, the protein is selected from the group consisting of thrombopoietin, erythropoietin, interferon, and soluble tumor necrosis factor receptor-1.
The present disclosure further provides methods for producing a variant protein having reduced allergenicity comprising the steps of: a) obtaining a naturally-occurring protein, wherein the naturally-occurring protein is selected from the group consisting of cytokines and cytokine receptors and preparing fragments of the naturally-occurring protein; b) contacting the fragments of the naturally-occurring protein with a first solution comprising naïve human CD4+ or CD8+ T-cells and differentiated dendritic cells; c) identifying an epitope region of the naturally-occurring protein, wherein the identifying step comprises measuring the ability of the fragments of the naturally-occurring protein epitope region to stimulate proliferation of the naïve human CD4+ or CD8+ T-cells; and d) modifying at least one amino acid in the epitope region identified in step c), to produce the variant protein. The methods further comprise the step of comparing the ability of the fragments of the naturally-occurring protein to stimulate proliferation of the naïve human CD4+ or CD8+ T-cells with the ability of the fragments of the variant protein to stimulate proliferation of the naïve human CD4+ or CD8+ T-cells. In some preferred embodiments, the protein is selected from the group consisting of thrombopoietin, erythropoietin, interferon, and soluble tumor necrosis factor receptor-1.
It is not intended that any of the methods of the present disclosure be conducted in any particular order, as far as preparation of pepsets and differentiation of dendritic cells. For example, the pepsets are prepared before the dendritic cells are differentiated, while in other embodiments, the dendritic cells are differentiated before the pepsets are prepared, and in still other embodiments, the dendritic cells are differentiated and the pepsets are prepared concurrently. Thus, it is not intended that the present disclosure be limited to methods having these steps in any particular order.
The present invention provides T-cell epitopes of IFN-β. These epitopes are provided in various sequences set forth herein. Indeed, in some particularly preferred embodiments, the present invention provides variant sequences (*i.e*., epitopes) suitable for substitution into IFN-β, including, the sequence set forth in SEQ ID NO:41.
The present invention provides T-cell epitopes of TPO These epitopes are provided in various sequences set forth herein. Indeed, the present disclosure provides variant sequences (*i.e*., epitopes) suitable for substitution into TPO, including, but not limited to the sequence set forth in SEQ ID NO:105.
The present disclosure provides T-cell epitopes of sTNF-R1. These epitopes are provided in various sequences set forth herein, Indeed, in some particularly preferred embodiments, the present disclosure provides variant sequences (*i.e*., epitopes) suitable for substitution into sTNF-R1, including, but not limited to the sequence set forth in SEQ ID NO:107.
The present disclosure provides T-cell epitopes of EPO These epitopes are provided in various sequences set forth herein. Indeed, the present disclosure provides variant sequences (*i.e*., epitopes) suitable for substitution into EPO, including, but not limited to the sequences set forth in SEQ ID NO:109 and SEQ ID NO:110.
The present disclosure provides I-MUNE® assay systems for identification of T-cell epitopes and T-cell non-epitopes, including but not limited to methods having the steps of combining differentiated dendritic cells with human CD4+ and/or CD8+ T-cells and with a peptide of interest (*e.g*., peptides derived from IFN- β, TPO or sTNF-R1). More specifically, peptides of interest that produce a reduced immunogenic response are provided, wherein a T-cell epitope is recognized comprising the steps of: (a) obtaining from a single blood source a solution of dendritic cells and a solution of CD4+ and/or CD8+ T-cells; (b) promoting differentiation in of the dendritic cells; (c) combining the solution of differentiated dendritic cells, CD4+ cells and/or CD8+ T-cells with a peptide of interest (*e.g*., a peptide comprising at least a portion of IFN-β, TPO and/or TNF-R1); and (d) measuring the proliferation of the T-cells in step (c).
In an example of the disclosure a series of peptide oligomers that correspond to all or parts of the cytokine or cytokine receptor are prepared. For example, a peptide library is produced covering the relevant portion or all of the IFN-β, TPO, or TNF-R1 protein. In one example, the manner of producing the peptides is to introduce overlap into the peptide library, for example, producing a first peptide corresponds to amino acid sequence 1-15 of the cytokine or cytokine receptor, a second peptide corresponds to amino acid sequence 4-18 of the cytokine or cytokine receptor, a third peptide corresponds to amino acid sequence 7-21 of cytokine or cytokine receptor, a fourth peptide corresponds to amino acid sequence 10-24 of the cytokine or cytokine receptor, etc. until representative peptides corresponding to the entire cytokine or cytokine receptor molecule are created. By analyzing each of the peptides individually in the I-MUNE® assay provided herein, it is possible to precisely identify the location of epitopes recognized by T-cells. In the example above, the greater reaction of one specific peptide than its neighbors facilitates identification of the epitope anchor region to within three amino acids. After determining the location of these epitopes, it is possible to alter the amino acids within each epitope until the peptide produces a different T-cell response from that of the original protein. Moreover, the present disclosure provides means for the identification of proteins that have desired low T-cell epitope potency that may be used in their naturally occurring forms.
Various *in vitro* and *in vivo* assays known in the art may be used to ascertain the reduced immunogenic response of variant proteins. *In vivo* assays include, but are not limited to HLA-DR3/DQ2 mouse T-cell responses, while suitable *in vitro* assays include, but are not limited to human peripheral blood mononuclear cell (PBMC) assays (See, Herman et al., J. Immunol.. 163:6275-6282 [1999]; Sonderstrup et al., Immunol. Rev., 172: 335-343 [1999]; Taneja and David, Immunol. Rev., 169:67-79 [1999]; and Grusby et al., Proc. Natl. Acad. Sci, 90:3913-3917 [1993]).
The present disclosure further provides cytokine and cytokine receptor compositions with reduced immunogenicity. In particular, the present invention provides such compositions that comprise epitopes described herein that reduce the immunogenic response to IFN-β. The present disclosure provides compositions that find use in various combinations of variant cytokines and/or cytokine receptors, as well as combinations that include wild-type proteins.
The present disclosure further provides compositions comprising nucleic acids sequences encoding variant cytokines and cytokine receptors, as well as expression vectors that comprise the nucleic acid, and host cells transformed with the expression vectors.
The present disclosure still further provides industrial and consumer-related products, including, but not limited to such compositions as personal care, health care, and cleansing compositions that comprise the variant cytokines and cytokine receptors of the present invention. Indeed, the present disclosure provides variant cytokines and cytokine receptors suitable for use in various applications, settings, and compositions.

### BRIEF DESCRIPTION OF THE DRAWINGS

Figure 1 provides the amino acid sequences (SEQ ID NOS:2-53) of the peptides synthesized based on the sequence of SEQ ID NO: 1.
Figure 2 provides I-MUNE® assay results for the peptides set forth in Figure 1.
Figure 3 provides the amino acid sequences (SEQ ID NOS:54-103) of a set of variant peptides based on the sequences of peptides #1 (SEQ ID NO:2), #27 (SEQ ID NO:28), and #40 (SEQ ID NO:41).
Figure 4 provides data showing the proliferative responses obtained for the variant peptides (*i.e*., those set forth in Figure 3). Panel A provides the percent response for each peptide, while Panel B provides the Stimulation Index for each peptide indicated.
Figure 5 provides data showing the proliferative responses obtained for the variant peptides of peptides #27 (SEQ ID NO:28) (Panel A) and #1 (SEQ ID NO:2) (Panel B).
Figure 6 provides data showing the magnitude of the proliferative response to peptide #40 (SEQ ID NO:93) in responders (top graph) and non-responders (bottom graph) expressing HLA-DQ6.
Figure 7 provides a graph showing the data obtained in the I-MUNE® assay with the first 152 residues of TPO (*i.e.,* the active domain).
Figure 8 provides a graph showing the results for the I-MUNE® assay conducted using the entire TPO molecule, including the CHO binding domain.
Figure 9, Panels A and B provide graphs showing the results for TPO epitope #45, in which the critical residues were tested.
Figure 10 provides a graph showing the data obtained in the I-MUNE® assay with soluble human tumor necrosis factor receptor-1 (sTNFR-1). The arrow indicates the epitope of interest.
Figure 11 provides a graph showing the data obtained in the I-MUNE® assay with TPO.

### DESCRIPTION OF THE INVENTION

The present disclosure provides means and compositions suitable for reducing the immunogenicity of cytokines and cytokines receptors such as interferon-β.
The present disclosure provides methods for the identification of CD4⁺ T-cell epitopes in the IFN-β, TPO, and/or soluble TNF-R1 protein sequence and the production of peptides that are no longer capable of initiating the CD4⁺T-cell response.
The development of an antibody to a protein requires as series of events that begin with a peptide segment derived from that protein being presented on the surface of a professional (activated) antigen presenting cell (APC). The peptide is associated with a specific protein on the surface of the antigen presenting cell, namely a protein in the major histocompatibility complex (MHC) (in humans, the MHC is referred to as the "human leukocyte antigen" (HLA) system). The bound peptide is capable of interacting with a second cell type, the T-cell. Specifically, the T-cell is of the subtype recognized by the expression of the CD4 protein on its surface (*i.e*., a CD4⁺T-cell). If the interaction is successful, the specific CD4⁺T-cell grows and divides (*i.e*., proliferates) and becomes capable of interacting with an antibody producing cell (*i.e*., a B-cell). If that interaction is successful, the B cell proliferates and becomes a center for the production of antibodies that are specifically directed against the original protein. Thus, the ultimate production of an antibody is dependent on the initial activation of a CD4⁺T-cell that is specific for a single peptide sequence (*i.e*., an epitope). Using the compositions and methods described herein, means are provided to predict which peptides from a target protein will be capable of the initial activation of specific CD4⁺ T-cells.
Application of recombinant DNA technology facilitates the rapid manipulation of protein or peptide sequences by changing the DNA sequence coding for the protein or peptide (*i.e*., a protein or peptide of interest). Application of this strategy to the gene coding for a target protein such as a cytokine or cytokine receptor facilitates the changing of the sequence of the epitopes, such that they are no longer capable of activating CD4⁺ T-cells. Such changes reduce the propensity of the cytokine or cytokine receptor to result in an antibody (Bab or Nab) response in humans. Thus, the present disclosure provides compositions and methods for the identification of CD4⁺ T-cell epitopes in cytokine and cytokine receptor protein sequences, and production of peptides which are no longer capable of initiating the CD4⁺T-cell response which, when incorporated through recombinant DNA technology, into the cytokine or cytokine receptor, reduce the capability of the cytokine (now a variant cytokine) or cytokine receptor (now a variant cytokine receptor) to initiate the formation of antibodies.

### Definitions

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionaries of many of the terms used in the invention. Although any methods and materials similar or equivalent to those described herein find use in the practice of the present disclosure, the preferred methods and materials are described herein. Accordingly, the terms defined immediately below are more fully described by reference to the Specification as a whole. Also, as used herein, the singular "a", "an" and "the" includes the plural reference unless the context clearly indicates otherwise. To facilitate understanding of the invention, a number of terms are defined below.
As used herein, the term "cytokine" refers to the soluble mediators that control many critical interactions among cells of the immune system. Cytokines comprise a diverse group of intercellular signaling peptides and glycoproteins. Most are genetically and structurally similar to each other. Each cytokine is secreted by a particular cell type in response to a variety of stimuli and produces characteristic effects on the growth, mobility, differentiation, and/or function of target cells. Collectively, cytokines regulate not only immune and inflammatory systems, but also are involved in wound healing, hematopoiesis, angiogenesis, and many other processes. It is intended that the term encompass all of the various cytokines, regardless of their structure, and commonly used nomenclature. For example, it is intended that the term encompass "lymphokines" (*i.e*., cytokines produced by lymphocytes), as well as "monokines" (*i.e*., cytokines produced by monocytes).
As used herein, "cytokine receptor" refers to receptor molecules that recognize and bind to cytokines. It is intended that the term encompass soluble cytokine receptors as well as cytokine receptors that are cell-bound. In some preferred embodiments, the term refers to soluble tumor necrosis factor receptor-1, which binds to excess tumor necrosis factor that is produced during various pathological conditions and exacerbates some diseases. It is intended that the term also encompass modified cytokine receptor molecules (*i.e*., "variant cytokine receptors"), including those with substitutions, deletions, and/or additions to the cytokine receptor amino acid and/or nucleic acid sequence. Thus, it is intended that the term encompass wild-type, as well as recombinant, synthetically-produced, and variant cytokine receptors.
The term "interferon-β," as used herein, refers to one member of a large class of secretory proteins that exhibit anti-viral activity, inhibit proliferation of vertebrate cells, and modulate immune responses.
As used herein, "thrombopoietin" ("TPO") refers to a cytokine that supports the growth and survival of hematopoietic stem cells (HSCs) and promotes platelet formation. Indeed, TPO is one of the hematopoietic colony-stimulating factors (CSFs) that support the production of particular mature blood cell types from pluripotent stem cells or committed progenitors in the bone marrow.
As used herein, "erythropoietin" (EPO") refers to a cytokine that enhances the production of erythrocyte (*i.e*., red blood cell) colonies. Like, TPO, EPO is also an important hematopoietic colony stimulating factor.
As used herein, "hematopoietin receptor family" refers to the family of proteins that recognize various hematopoietic factors.
As used herein, "interleukin" ("IL") refers to a group of cytokines produced by various cells, that have numerous and varied effects on the immune system, inflammation, fever, hematopoiesis, thrombopoiesis, proliferation of lymphocytes, expression of immunoglobulins, acute phase response, activation, growth and function of various polymorphonuclear cells, etc. It is intended that the term encompass any interleukins (*e.g*., IL-1, IL-2, IL-3, IL-4, IL-5, etc.).
As used herein, "tumor necrosis factor" ("TNF") refers to a cytokine that promotes growth and differentiation of B-cells, activation of neutrophils and macrophages, stimulates hematopoiesis, and produces a broad range of effects on non-hematopoietic cells, as well as inducing the expression of many other cytokines and mediators that promote inflammation, and enhances activation of T-helper cells by antigen presenting cells.
"Antigen presenting cell" as used herein refers to cells of the immune system which present antigen on their surface that is recognizable by T-cells. Examples of antigen presenting cells are dendritic cells, interdigitating cells, activated B-cells and macrophages.
The term "lymphoid" when used in reference to a cell line or a cell, means that the cell line or cell is derived from the lymphoid lineage and includes cells of both the B and the T lymphocyte lineages.
As used herein, the terms "T lymphocyte" and "T-cell," encompass any cell within the T lymphocyte lineage from T-cell precursors (including Thy1 positive cells which have not rearranged the T cell receptor genes) to mature T cells (*i.e*., single positive for either CD4 or CD8, surface TCR positive cells).
As used herein, the terms "B lymphocyte" and "B-cell" encompasses any cell within the B-cell lineage from B-cell precursors, such as pre-B-celis (B220⁺ cells which have begun to rearrange Ig heavy chain genes), to mature B-cells and plasma cells.
As used herein, "CD4⁺ T-cell" and "CD4 T-cell" refer to helper T-cells, while "CD8⁺ T-cell" and CD8 T-cell" refer to cytotoxic T-cells.
As used herein, "B-cell proliferation," refers to the number of B-cells produced during the incubation of B-cells with the antigen presenting cells, with or without antigen.
As used herein, "baseline B-cell proliferation," as used herein, refers to the degree of B-cell proliferation that is normally seen in an individual in response to exposure to antigen presenting cells in the absence of peptide or protein antigen. For the purposes herein, the baseline B-cell proliferation level is determined on a per sample basis for each individual as the proliferation of B-cells in the absence of antigen.
As used herein, "B-cell epitope," refers to a feature of a peptide or protein that is recognized by a B-cell receptor in the immunogenic response to the peptide comprising that antigen (*i.e*., the immunogen).
As used herein, "altered B-cell epitope," refers to an epitope amino acid sequence which differs from the precursor peptide or peptide of interest, such that the variant peptide of interest produces different (*i.e*., altered) immunogenic responses in a human or another animal. It is contemplated that an altered immunogenic response includes altered immunogenicity and/or allergenicity (*i.e*., an either increased or decreased overall immunogenic response). In some embodiments, the altered B-cell epitope comprises substitution and/or deletion of an amino acid selected from those residues within the identified epitope. In alternative embodiments, the altered B-cell epitope comprises an addition of one or more residues within the epitope.
As used herein "T-cell epitope" means a feature of a peptide or protein that is recognized by a T-cell receptor in the initiation of an immunologic response to the peptide comprising that antigen. Recognition of a T-cell epitope by a T-cell is generally believed to be via a mechanism wherein T-cells recognize peptide fragments of antigens which are bound to class I or class II Major Histocompatibility Complex (MHC) molecules expressed on antigen-presenting cells (*See e.g.,* Moeller (ed.), Immunol. Rev., 98:187 [1987]). In some embodiments of the present invention, the epitopes or epitopic fragments identified as described herein find use in the detection of antigen presenting cells having MHC molecules capable of binding and displaying the epitopes or fragments. In some embodiments, the epitopes/epitopic fragments further comprise a detectable label (*i.e*., a marker) that facilitates the identification of cells that bind and/or display the epitope/epitopic fragment of interest.
As used herein, "T-cell proliferation," refers to the number of T-cells produced during the incubation of T-cells with the antigen presenting cells, with or without antigen.
"Baseline T-cell proliferation," as used herein, refers to the degree of T-cell proliferation that is normally seen in an individual in response to exposure to antigen presenting cells in the absence of peptide or protein antigen. For the purposes herein, the baseline T-cell proliferation level is determined on a per sample basis for each individual as the proliferation of T-cells in response to antigen presenting cells in the absence of antigen.
As used herein "altered immunogenic response," refers to an increased or reduced immunogenic response. Proteins and peptides exhibit an "increased immunogenic response" when the T-cell and/or B-cell response they evoke is greater than that evoked by a parental (*e.g*., precursor) protein or peptide (*e.g*., the protein of interest). The net result of this higher response is an increased antibody response directed against the variant protein or peptide. Proteins and peptides exhibit a "reduced immunogenic response" when the T-cell and/or B-cell response they evoke is less than that evoked by a parental (*e.g*., precursor) protein or peptide. In preferred embodiments, the net result of this lower response is a reduced antibody response directed against the variant protein or peptide. In some preferred embodiments, the parental protein is a wild-type protein or peptide.
As used herein, the term "significant epitope" refers to an epitope (*i.e*., a T-cell and/or B-cell epitope) wherein the response rate within the tested donor pool is equal to or greater than about three times the background response rate.
As used herein, a "weakly significant epitope" refers to an epitope (*i.e*., a T-cell and/or B-cell epitope), wherein the response rate within the tested donor pool is greater than the background response rate, but less than about three times the background rate.
As used herein, "background level" and "background response" refer to the average percent of responders to any given peptide in the dataset for any tested protein. This value is determined by averaging the percent responders for all peptides in the set, as compiled for all the tested donors. As an example, a 3% background response would indicate that on average there would be three positive (SI greater than 2.95) responses for any peptide in a dataset when tested on 100 donors.
The term "sample" as used herein is used in its broadest sense. However, in preferred embodiments, the term is used in reference to a sample (*e.g*., an aliquot) that comprises a peptide (*i.e*., a peptide within a pepset, that comprises a sequence of a protein of interest) that is being analyzed, identified, modified, and/or compared with other peptides. Thus, in most cases, this term is used in reference to material that includes a protein or peptide that is of interest.
As used herein, "protein of interest," refers to a protein which is being analyzed, identified and/or modified. Naturally-occurring, as well as recombinant proteins, synthetically produced, variant and derivative proteins, all find use in the present invention.
As used herein, "protein" refers to any composition comprised of amino acids and recognized as a protein by those of skill in the art. The terms "protein," "peptide" and polypeptide are used interchangeably herein. Amino acids may be referred to by their complete names (*e.g*., alanine) or by the accepted one letter (*e.g*., A), or three letter (*e.g*., ala) abbreviations. Wherein a peptide is a portion of a protein, those skill in the art understand the use of the term in context. The term "protein" encompasses mature forms of proteins, as well as the pro- and prepro-forms of related proteins. Prepro forms of proteins comprise the mature form of the protein having a prosequence operably linked to the amino terminus of the protein, and a "pre-" or "signal" sequence operably linked to the amino terminus of the prosequence.
As used herein, functionally similar proteins are considered to be "related proteins." In some embodiments, these proteins are derived from a different genus and/or species, including differences between classes of organisms (*e.g*., a bacterial protein and a fungal protein). In additional embodiments, related proteins are provided from the same species. Indeed, it is not intended that the present invention be limited to related proteins from any particular source(s).
As used herein, the term "derivative" refers to a protein which is derived from a precursor protein by addition of one or more amino acids to either or both the C- and N-terminal end(s), substitution of one or more amino acids at one or a number of different sites in the amino acid sequence, and/or deletion of one or more amino acids at either or both ends of the protein or at one or more sites in the amino acid sequence, and/or insertion of one or more amino acids at one or more sites in the amino acid sequence.
The preparation of a protein derivative is preferably achieved by modifying a DNA sequence which encodes for the native protein, transformation of that DNA sequence into a suitable host, and expression of the modified DNA sequence to form the derivative protein.
One type of related (and derivative) proteins are "variant proteins." In preferred embodiments, variant proteins differ from a parent protein and one another by a small number of amino acid residues. The number of differing amino acid residues may be one or more, preferably 1, 2, 3, 4, 5, 10, 15, 20, 30, 40, 50, or more amino acid residues. In one preferred embodiment, the number of different amino acids between variants is between 1 and 10. In particularly preferred embodiments, related proteins and particularly variant proteins comprise at least 50%, 60%, 65%. 70%, 75%, 80%, 85%, 90%, 95%, 97%, 98%, or 99% amino acid sequence identity. Additionally, a related protein or a variant protein as used herein, refers to a protein that differs from another related protein or a parent protein in the number of prominent regions. For example, variant proteins have 1, 2, 3, 4, 5, or 10 corresponding prominent regions that differ from the parent protein.
In one example, the prominent corresponding region of a variant produces only a background level of immunogenic response. Some of the residues identified for substitution, insertion or deletion are conserved residues whereas others are not. In the case of residues which are not conserved, the replacement of one or more amino acids is limited to substitutions which produce a variant which has an amino acid sequence that does not correspond to one found in nature. In the case of conserved residues, such replacements should not result in a naturally-occurring sequence.
The following cassette mutagenesis method may be used to facilitate the construction of the protein variants of the present invention, although other methods may be used. First, the naturally-occurring gene encoding the protein is obtained and sequenced in whole or in part. Then the sequence is scanned for a point at which it is desired to make a mutation (deletion, insertion or substitution) of one or more amino acids in the encoded protein. The sequences flanking this point are evaluated for the presence of restriction sites for replacing a short segment of the gene with an oligonucleotide pool which when expressed will encode various mutants. Such restriction sites are preferably unique sites within the protein gene so as to facilitate the replacement of the gene segment. However, any convenient restriction site which is not overly redundant in the protein gene may be used, provided the gene fragments generated by restriction digestion can be reassembled in proper sequence. If restriction sites are not present at locations within a convenient distance from the selected point (from 10 to 15 nucleotides), such sites are generated by substituting nucleotides in the gene in such a fashion that neither the reading frame nor the amino acids encoded are changed in the final construction. Mutation of the gene in order to change its sequence to conform to the desired sequence is accomplished by M13 primer extension in accord with generally known methods. The task of locating suitable flanking regions and evaluating the needed changes to arrive at two convenient restriction site sequences is made routine by the redundancy of the genetic code, a restriction enzyme map of the gene and the large number of different restriction enzymes. Note that if a convenient flanking restriction site is available, the above method need be used only in connection with the flanking region which does not contain a site.
Once the naturally-occurring DNA or synthetic DNA is cloned, the restriction sites flanking the positions to be mutated are digested with the cognate restriction enzymes and a plurality of end termini-complementary oligonucleotide cassettes are ligated into the gene. The mutagenesis is simplified by this method because all of the oligonucleotides can be synthesized so as to have the same restriction sites, and no synthetic linkers are necessary to create the restriction sites.
As used herein, "corresponding to," refers to a residue at the enumerated position in a protein or peptide, or a residue that is analogous, homologous, or equivalent to an enumerated residue in a protein or peptide.
As used herein, "corresponding region" generally refers to an analogous position along related proteins or a parent protein.
As used herein, the term "analogous sequence" refers to a sequence within a protein that provides similar function, tertiary structure, and/or conserved residues as the protein of interest (*i.e*., typically the original protein of interest). In particularly preferred embodiments, the analogous sequence involves sequence(s) at or near an epitope. For example, in epitope regions that contain an alpha helix or a beta sheet structure, the replacement amino acids in the analogous sequence preferably maintain the same specific structure. The term also refers to nucleotide sequences, as well as amino acid sequences. In some embodiments, analogous sequences are developed such that the replacement amino acids show a similar function, the tertiary structure and/or conserved residues to the amino acids in the protein of interest at or near the epitope. Thus, where the epitope region contains, for example, an alpha-helix or a beta-sheet structure, the replacement amino acids preferably maintain that specific structure.
As used herein, "homologous protein" refers to a protein that has similar action, structure, antigenic, and/or immunogenic response as the protein of interest. It is not intended that a homolog and a protein of interest be necessarily related evolutionarily. Thus, it is intended that the term encompass the same functional protein obtained from different species. In some preferred examples, it is desirable to identify a homolog that has a tertiary and/or primary structure similar to the protein of interest, as replacement for the epitope in the protein of interest with an analogous segment from the homolog will reduce the disruptiveness of the change. Thus, in most cases, closely homologous proteins provide the most desirable sources of epitope substitutions. Alternatively, it is advantageous to look to human analogs for a given protein. For example, in some embodiments, substituting a specific epitope in one human cytokine with a sequence from another cytokine or other species' cytokine results in the production of cytokine with reduced immunogenicity.
As used herein, "homologous genes" refers to at least a pair of genes from different, but usually related species, which correspond to each other and which are identical or very similar to each other. The term encompasses genes that are separated by speciation (*i.e*., the development of new species) (*e.g*., orthologous genes), as well as genes that have been separated by genetic duplication (*e.g*., paralogous genes). These genes encode "homologous proteins."
As used herein, "ortholog" and "orthologous genes" refer to genes in different species that have evolved from a common ancestral gene (*i.e*., a homologous gene) by speciation. Typically, orthologs retain the same function in during the course of evolution. Identification of orthologs finds use in the reliable prediction of gene function in newly sequenced genomes.
As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.
As used herein, "wild-type" and "native" proteins are those found in nature. The terms "wild-type sequence," and "wild-type gene" are used interchangeably herein, to refer to a sequence that is native or naturally occurring in a host cell. In some examples, the wild-type sequence refers to a sequence of interest that is the starting point of a protein engineering project. The genes encoding the naturally-occurring (*i.e*., precursor) protein may be obtained in accord with the general methods known to those skilled in the art. The methods generally comprise synthesizing labeled probes having putative sequences encoding regions of the protein of interest, preparing genomic libraries from organisms expressing the protein, and screening the libraries for the gene of interest by hybridization to the probes. Positively hybridizing clones are then mapped and sequenced.
The term "recombinant DNA molecule" as used herein refers to a DNA molecule that is comprised of segments of DNA joined together by means of molecular biological techniques.
The degree of homology between sequences may be determined using any suitable method known in the art (*See e.g.,* Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, WI); and Devereux et al., Nucl. Acid Res., 12:387-395 [1984]).
For example, PILEUP is a useful program to determine sequence homology levels. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle, (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps. Another example of a useful algorithm is the BLAST algorithm, described by Altschul *et al.,* (Altschul et al., J. Mol. Biol., 215:403-410, [1990]; and Karlin et al., Proc. Natl. Acad. Sci. USA 90:5873-5787 [1993]). One particularly useful BLAST program is the WU-BLAST-2 program (See, Altschul et al., Meth. Enzymol." 266:460-480 [1996]). parameters "W," "T," and "X" determine the sensitivity and speed of the alignment. The BLAST program uses as defaults a wordlength (W) of 11, the BLOSUM62 scoring matrix (See, Henikoff and Henikoff, Proc. Natl. Acad. Sci. USA 89:10915 [1989]) alignments (B) of 50, expectation (E) of 10, M'5, N'-4, and a comparison of both strands.
As used herein, "percent (%) nucleic acid sequence identity" is defined as the percentage of nucleotide residues in a candidate sequence that are identical with the nucleotide residues of the sequence.
As used herein, the term "hybridization" refers to the process by which a strand of nucleic acid joins with a complementary strand through base pairing, as known in the art.
As used herein, "maximum stringency" refers to the level of hybridization that typically occurs at about Tm-5°C (5°C below the Tm of the probe); "high stringency" at about 5°C to 10°C below Tm; "intermediate stringency" at about 10°C to 20°C below Tm; and "low stringency" at about 20°C to 25°C below Tm. As will be understood by those of skill in the art, a maximum stringency hybridization can be used to identify or detect identical polynucleotide sequences while an intermediate or low stringency hybridization can be used to identify or detect polynucleotide sequence homologs.
The phrases "substantially similar and "substantially identical" in the context of two nucleic acids or polypeptides typically means that a polynucleotide or polypeptide comprises a sequence that has at least 75% sequence identity, preferably at least 80%, more preferably at least 90%, still more preferably 95%, most preferably 97%, sometimes as much as 98% and 99% sequence identity, compared to the reference (*i.e*., wild-type) sequence. Sequence identity may be determined using known programs such as BLAST, ALIGN, and CLUSTAL using standard parameters. (See *e.g*., Altschul, et al., J. Mol. Biol. 215:403-410 [1990]; Henikoff et al., Proc. Natl. Acad Sci. USA 89:10915 [1989]; Karin et al., Proc. Natl Acad. Sci USA 90:5873 [1993]; and Higgins et al., Gene 73:237 - 244 [1988]). Software for performing BLAST analyses is publicly available through the National Center for Biotechnology Information. Also, databases may be searched using FASTA (Pearson et al., Proc. Natl. Acad. Sci. USA 85:2444-2448 [1988]).
As used herein, "equivalent residues" refers to proteins that share particular amino acid residues. For example, equivalent resides may be identified by determining homology at the level of tertiary structure for a protein (*e.g*. IFN-β) whose tertiary structure has been determined by x-ray crystallography. Equivalent residues are defined as those for which the atomic coordinates of two or more of the main chain atoms of a particular amino acid residue of the protein having putative equivalent residues and the protein of interest (N on N, CA on CA, C on C and O on O) are within 0.13 nm and preferably 0.1 nm after alignment. Alignment is achieved after the best model has been oriented and positioned to give the maximum overlap of atomic coordinates of non-hydrogen protein atoms of the proteins analyzed. The preferred model is the crystallographic model giving the lowest R factor for experimental diffraction data at the highest resolution available, determined using methods known to those skilled in the art of crystallography and protein characterization/analysis.
In some examples, modification is preferably made to the "precursor DNA sequence" which encodes the amino acid sequence of the precursor enzyme, but can be by the manipulation of the precursor protein. In the case of residues which are not conserved, the replacement of one or more amino acids is limited to substitutions which produce a variant which has an amino acid sequence that does not correspond to one found in nature. In the case of conserved residues, such replacements should not result in a naturally-occurring sequence. Derivatives provided by the present invention further include chemical modification(s) that change the characteristics of the protease.
In some preferred embodiments, the protein gene is ligated into an appropriate expression plasmid. The cloned protein gene is then used to transform or transfect a host cell in order to express the protein gene. This plasmid may replicate in hosts in the sense that it contains the well-known elements necessary for plasmid replication or the plasmid may be designed to integrate into the host chromosome. The necessary elements are provided for efficient gene expression (*e.g*., a promoter operably linked to the gene of interest). In some examples, these necessary elements are supplied as the gene's own homologous promoter if it is recognized, (*i.e*., transcribed, by the host), a transcription terminator (a polyadenylation region for eukaryotic host cells) which is exogenous or is supplied by the endogenous terminator region of the protein gene. In some examples, a selection gene such as an antibiotic resistance gene that enables continuous cultural maintenance of plasmid-infected host cells by growth in antimicrobial-containing media is also included.
The present disclosure encompasses proteins having altered immunogenicity that are equivalent. Being "equivalent," means that the proteins are encoded by a polynucleotide capable of hybridizing to the polynucleotide having the sequence as shown in any one of those provided herein, under conditions of medium to high stringency and still retaining the altered immunogenic response to human T-cells. Being "equivalent" means that the protease comprises at least 55%, at least 65%, at least 70%, at least 75%, at least 80%, at least 85%, at least 90%, at least 95%, at least 97% or at least 99% identity to the epitope sequences and the variant proteases having such epitopes (*e.g*., having the amino acid sequence modified).
As used herein, the terms "hybrid proteins" and "fusion proteins " refer to proteins that are engineered from at least two different or "parental" proteins. In preferred examples, these parental proteins are homologs of one another. For example, in some embodiments, a preferred hybrid protease or fusion protein contains the N-terminus of a protein and the C-terminus of a homolog of the protein. In some preferred examples, the two terminal ends are combined to correspond to the full-length active protein. In alternative preferred examples, the homologs share substantial similarity but do not have identical T-cell epitopes. Therefore, in one example, the present disclosure provides a protease of interest having one or more T-cell epitopes in the C-terminus, but in which the C-terminus is replaced with the C-terminus of a homolog having a less potent T-cell epitope, or fewer or no T-cell epitopes in the C-terminus. Thus, the skilled artisan understands that by being able to identify T-cell epitopes among homologs, a variety of variants producing different immunogenic responses can be formed. Moreover, it is understood that internal portions, and more than one homolog can be used to produce the variants of the present disclosure.
"Operably linked" and "in operable combination," when describing the relationship between two DNA regions, simply means that they are functionally related to each other. For example, a presequence is operably linked to a peptide if it functions as a signal sequence, participating in the secretion of the mature form of the protein most probably involving cleavage of the signal sequence. A promoter is operably linked to a coding sequence if it controls the transcription of the sequence; a ribosome binding site is operably linked to a coding sequence if it is positioned so as to permit translation.
DNA molecules are said to have "5' ends" and "3' ends" because mononucleotides are reacted to make oligonucleotides in a manner such that the 5' phosphate of one mononucleotide pentose ring is attached to the 3' oxygen of its neighbor in one direction via a phosphodiester linkage. Therefore, an end of an oligonucleotides is referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide, also may be said to have 5' and 3' ends. In either a linear or circular DNA molecule, discrete elements are referred to as being "upstream" or 5' of the "downstream" or 3' elements. This terminology reflects the fact that transcription proceeds in a 5' to 3' fashion along the DNA strand. The promoter and enhancer elements which direct transcription of a linked gene are generally located 5' or upstream of the coding region (enhancer elements can exert their effect even when located 3' of the promoter element and the coding region). Transcription termination and polyadenylation signals are located 3' or downstream of the coding region.
The term "an oligonucleotide having a nucleotide sequence encoding a gene" means a DNA sequence comprising the coding region of a gene or, in other words, the DNA sequence that encodes a gene product. The coding region may be present in either a cDNA or genomic DNA form. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, etc. or a combination of both endogenous and exogenous control elements.
The term "recombinant oligonucleotide" refers to an oligonucleotide created using molecular biological manipulations, including but not limited to, the ligation of two or more oligonucleotide sequences generated by restriction enzyme digestion of a polynucleotide sequence, the synthesis of oligonucleotides (*e.g*., the synthesis of primers or oligonucleotides) and the like.
The term "transcription unit" as used herein refers to the segment of DNA between the sites of initiation and termination of transcription and the regulatory elements necessary for the efficient initiation and termination. For example, a segment of DNA comprising an enhancer/promoter, a coding region, and a termination and polyadenylation sequence comprises a transcription unit.
The term "regulatory element" as used herein refers to a genetic element that controls some aspect of the expression of nucleic acid sequences. For example, a promoter is a regulatory element which facilitates the initiation of transcription of an operably linked coding region. Other regulatory elements are splicing signals, polyadenylation signals, termination signals, etc. (defined *infra*).
The term "expression vector" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes include a promoter, optionally an operator sequence, a ribosome binding site and possibly other sequences. Eukaryotic cells are known to utilize promoters, enhancers, and termination and polyadenylation signals. Once transformed into a suitable host, the vector may replicate and function independently of the host genome, or may, in some instances, integrate into the genome itself. In the present specification, "plasmid" and "vector" are sometimes used interchangeably as the plasmid is the most commonly used form of vector at present. However, it is intended to include such other forms of expression vectors which serve equivalent functions and which are, or become, known in the art, including but not limited to plasmids, phage particles, or simply potential genomic inserts.
The "host cells" used in the present disclosure generally are prokaryotic or eukaryotic hosts which contain an expression vector and/or gene of interest. Host cells are transformed or transfected with vectors constructed using recombinant DNA techniques. Such transformed host cells are capable of either replicating vectors encoding the protein variants or expressing the desired protein variant. In the case of vectors which encode the pre- or prepro-form of the protein variant, such variants, when expressed, are typically secreted from the host cell into the host cell medium.
The term "promoter/enhancer" denotes a segment of DNA which contains sequences capable of providing both promoter and enhancer functions (for example, the long terminal repeats of retroviruses contain both promoter and enhancer functions). The enhancer/promoter may be "endogenous" or "exogenous" or "heterologous." An endogenous enhancer/promoter is one which is naturally linked with a given gene in the genome. An exogenous (heterologous) enhancer/promoter is one which is placed in juxtaposition to a gene by means of genetic manipulation (*i.e*., molecular biological techniques).
The presence of "splicing signals" on an expression vector often results in higher levels of expression of the recombinant transcript. Splicing signals mediate the removal of introns from the primary RNA transcript and consist of a splice donor and acceptor site (Sambrook et al., Molecular Cloning: A Laboratory Manual, 2nd ed., Cold Spring Harbor Laboratory Press, New York [1989], pp. 16.7-16.8). A commonly used splice donor and acceptor site is the splice junction from the 16S RNA of SV40.
Efficient expression of recombinant DNA sequences in eukaryotic cells requires signals directing the efficient termination and polyadenylation of the resulting transcript. Transcription termination signals are generally found downstream of the polyadenylation signal and are a few hundred nucleotides in length. The term "poly A site" or "poly A sequence" as used herein denotes a DNA sequence which directs both the termination and polyadenylation of the nascent RNA transcript. Efficient polyadenylation of the recombinant transcript is desirable as transcripts lacking a poly A tail are unstable and are rapidly degraded. The poly A signal utilized in an expression vector may be "heterologous" or "endogenous." An endogenous poly A signal is one that is found naturally at the 3' end of the coding region of a given gene in the genome. A heterologous poly A signal is one which is isolated from one gene and placed 3' of another gene. A commonly used heterologous poly A signal is the SV40 poly A signal.
The terms "stable transfection" and "stably transfected" refer to the introduction and integration of foreign DNA into the genome of the transfected cell. The term "stable transfectant" refers to a cell which has stably integrated foreign DNA into the genomic DNA.
The terms "selectable marker" and "selectable gene product" as used herein refer to the use of a gene which encodes an enzymatic activity that confers resistance to an antibiotic or drug upon the cell in which the selectable marker is expressed.
As used herein, the terms "amplification" and "gene amplification" refer to a process by which specific DNA sequences are disproportionately replicated such that the amplified gene becomes present in a higher copy number than was initially present in the genome. In some embodiments, selection of cells by growth in the presence of a drug (*e.g*., an inhibitor of an inhibitable enzyme) results in the amplification of either the endogenous gene encoding the gene product required for growth in the presence of the drug or by amplification of exogenous (*i.e*., input) sequences encoding this gene product, or both. Gene amplification occurs naturally during development in particular genes such as the amplification of ribosomal genes in amphibian oocytes. Gene amplification may be induced by treating cultured cells with drugs. An example of drug-induced amplification is the methotrexate-induced amplification of the endogenous dhfrgene in mammalian cells (Schmike et al., Science 202:1051 [1978]). Selection of cells by growth in the presence of a drug (*e.g*., an inhibitor of an inhibitable enzyme) may result in the amplification of either the endogenous gene encoding the gene product required for growth in the presence of the drug or by amplification of exogenous (*i.e*., input) sequences encoding this gene product, or both.
Amplification is a special case of nucleic acid replication involving template specificity. It is to be contrasted with non-specific template replication (*i.e*., replication that is template-dependent but not dependent on a specific template). Template specificity is here distinguished from fidelity of replication (*i.e*., synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.
As used herein, the term "co-amplification" refers to the introduction into a single cell of an amplifiable marker in conjunction with other gene sequences (*i.e*., comprising one or more non-selectable genes such as those contained within an expression vector) and the application of appropriate selective pressure such that the cell amplifies both the amplifiable marker and the other, non-selectable gene sequences. The amplifiable marker may be physically linked to the other gene sequences or alternatively two separate pieces of DNA, one containing the amplifiable marker and the other containing the non-selectable marker, may be introduced into the same cell.
As used herein, the terms "amplifiable marker," "amplifiable gene," and "amplification vector" refer to a gene or a vector encoding a gene which permits the amplification of that gene under appropriate growth conditions.
As used herein, the term "amplifiable nucleic acid" refers to nucleic acids which may be amplified by any amplification method. It is contemplated that "amplifiable nucleic acid" will usually comprise "sample template."
As used herein, the term "sample template" refers to nucleic acid originating from a sample which is analyzed for the presence of "target" (defined below). In contrast, "background template" is used in reference to nucleic acid other than sample template which may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.
"Template specificity" is achieved in most amplification techniques by the choice of enzyme. Amplification enzymes are enzymes that, under conditions they are used, will process only specific sequences of nucleic acid in a heterogeneous mixture of nucleic acid. For example, in the case of Qβ replicase, MDV-1 RNA is the specific template for the replicase (See *e.g*., Kacian et al., Proc. Natl. Acad. Sci. USA 69:3038 [1972]). Other nucleic acids are not replicated by this amplification enzyme. Similarly, in the case of T7 RNA polymerase, this amplification enzyme has a stringent specificity for its own promoters (See, Chamberlin et al., Nature 228:227 [1970]). In the case of T4 DNA ligase, the enzyme will not ligate the two oligonucleotides or polynucleotides, where there is a mismatch between the oligonucleotide or polynucleotide substrate and the template at the ligation junction (See, Wu and Wallace, Genomics 4:560 [1989]). Finally, *Taq* and *Pfu* polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences.
As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e.,* in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.
As used herein, the term "probe" refers to an oligonucleotide (*i.e*., a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (*e.g*., ELISA, as well as enzyme-based histochemical assays), fluorescent, radioactive, and luminescent systems. It is not intended that the present disclosure be limited to any particular detection system or label.
As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.
As used herein, the term "polymerase chain reaction" ("PCR") refers to the methods of U.S. Patent Nos. 4,683,195 4,683,202, and 4,965,188, which include methods for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (*i.e*., denaturation, annealing and extension constitute one "code"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".
As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).
With PCR, it is possible to amplify a single copy of a specific target sequence in genomic DNA to a level detectable by several different methodologies (*e.g*., hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.
As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.
As used herein, the terms "restriction endonucleases" and "restriction enzymes" refer to bacterial enzymes, each of which cut double-stranded DNA at or near a specific nucleotide sequence.
The terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence
The present disclosure provides methods for the identification of CD4⁺ T-cell epitopes in the sequences of various human cytokines and cytokine receptors, as well as the production of peptides which when incorporated into the cytokine or cytokine receptor sequence, are no longer capable of initiating the CD4⁺ T-cell response. The present invention provides means and compositions suitable for reducing the immunogenicity of cytokines such as interferon-β (IFN-β).
In some examples, the present disclosure provides methods for the identification of CD4⁺ T-cell epitopes in the IFN-β, EPO, TPO, and/or soluble TNF-R1 protein sequence and the production of peptides that are no longer capable of initiating the CD4⁺ T-cell response. In particular, the present disclosure provides means and compositions suitable for reducing the immunogenicity of cytokines and cytokine receptors.
In these embodiments, the present disclosure provides means for determining the T-cell responses of humans against various epitopes comprising a protein of interest. In additional examples, once the significant epitopes are identified using the I-MUNE® assay system described herein, the significant epitopes are altered to produce epitopes that induce either a reduced or an enhance immune response to the protein.
Thus, as indicated above, the proteins of the present disclosure exhibit modified immunogenic responses (*e.g*., antigenicity and/or immunogenicity) when compared to the native proteins encoded by their precursor DNAs. In some preferred examples, the proteins exhibit reduced allergenicity. Those of skill in the art readily recognize that the uses of the proteases of this disclosure will be determined, in large part, on the immunological properties of the proteins. For example, cytokines and cytokine receptors that exhibit reduced immunogenic responses (*e.g*., variant cytokines and variant cytokine receptors) find use in treatment compositions. An effective amount of one or more cytokine variants and/or cytokine receptor variants described herein find use in compositions useful for treatment of diseases and inflammation. An effective amount of one or more related and/or variant proteins (*e.g*., cytokines and/or cytokine receptors) with reduced allergenicity/immunogenicity find use in various compositions that are suitable to replace currently used cytokines and cytokine receptors for treatment of various conditions.

### EXPERIMENTAL

The following examples serve to illustrate certain preferred examples and aspects of the present disclosure.
In the experimental disclosure which follows, the following abbreviations apply: M (molar); mM (millimolar); µM (micromolar); nM (nanomolar); mol (moles); mmol (millimoles); µmol (micromoles); nmol (nanomoles); gm (grams); mg (milligrams); µg (micrograms); pg (picograms); L (liters); ml (milliliters); µl (microliters); cm (centimeters); mm (millimeters); µm (micrometers); nm (nanometers); °C (degrees Centigrade); cDNA (copy or complimentary DNA); DNA (deoxyribonucleic acid); ssDNA (single stranded DNA); dsDNA (double stranded DNA); dNTP (deoxyribonucleotide triphosphate); RNA (ribonucleic acid); PBS (phosphate buffered saline); g (gravity); OD (optical density); Dulbecco's phosphate buffered solution (DPBS); HEPES (N-[2-Hydroxyethyl]piperazine-N-[2-ethanesulfonic acid]); HBS (HEPES buffered saline); SDS (sodium dodecylsulfate); Tris-HCl (tris[Hydroxymethyl]aminomethane-hydrochloride); Klenow (DNA polymerase I large (Klenow) fragment); rpm (revolutions per minute); EGTA (ethylene glycol-bis(β-aminoethyl ether) N, N, N', N'-tetraacetic acid); EDTA (ethylenediaminetetracetic acid); bla (β-lactamase or ampicillin-resistance gene); Endogen (Endogen, Woburn, MA); CytoVax (CytoVax, Edmonton, Canada); Wyeth-Ayerst (Wyeth-Ayerst, Philadelphia, PA); NEN (NEN Life Science Products, Boston, MA); Wallace Oy (Wallace Oy, Turku, Finland); Pharma AS (Pharma AS, Oslo, Norway); Dynal (Dynal, Oslo, Norway); Bio-Synthesis (Bio-Synthesis, Lewisville, TX); ATCC (American Type Culture Collection, Rockville, MD); Gibco/BRL (Gibco/BRL, Grand Island, NY); Sigma (Sigma Chemical Co., St. Louis, MO); Pharmacia (Pharmacia Biotech, Pisacataway, NJ); and Stratagene (Stratagene, La Jolla, CA).

### EXAMPLE 1

### Preparation of Cells Used in the I-MUNE® Assay System for the Identification of Peptide T-Cell Epitopes in IFN-β Using Human T-Cells

Fresh human peripheral blood cells were collected from 87 humans of unknown exposure status to IFN-β. These cells were tested to determine antigenic epitopes in IFN-β, as described in Example 3.
Peripheral mononuclear blood cells (stored at room temperature, no older than 24 hours) were prepared for use as follows: Approximately 30 mls of a solution of buffy coat preparation from one unit of whole blood was brought to 50 ml with Dulbecco's phosphate buffered solution (DPBS) and split into two tubes. The samples were underlaid with 12.5 ml of room temperature Lymphoprep density separation media (Nycomed; Pharma AS; Density 1.077 g/ml). The tubes were centrifuged for thirty minutes at 600 xg. The interface of the two phases was collected, pooled and washed in DPBS. The cell density of the resultant solution was measured by hemocytometer, as known in the art. Viability was measured by trypan blue exclusion, as known in the art.
From the resulting solution, a differentiated dendritic cell culture was prepared from the peripheral blood mononuclear cell sample having a density of 10⁸ cells per 75 ml culture flask in a solution as described below:
(1) 50 ml of serum free AIM V media (Gibco) was supplemented with a 1:100 dilution beta-mercaptoethanol (Gibco). The flasks were laid flat for two hours at 37°C in 5% CO₂ to allow adherence of monocytes to the flask wall.
(2) Differentiation of the monocyte cells to dendritic cells was performed as follows: nonadherent cells were removed and the resultant adherent cells (monocytes) combined with 30 ml of AIM V, 800 units/ml of GM-CSF (Endogen) and 500 units/ml of IL-4 (Endogen); the resulting mixture was cultured for 5 days at 37°C in 5% CO₂. After the five days of incubation, the cytokine TNFα (Endogen) was added to 0.2 units/ml, and the cytokine IL-1α (Endogen) was added to a final concentration of 50 units/ml and the mixture incubated at 37°C in 5% CO₂ for two more days.
(3) On the seventh day, mitomycin C was added to a concentration of 50 micrograms/ml in 100 mM EDTA-containing PBS to stop growth of the now differentiated dendritic cell culture. The solution was incubated for 60 minutes at 37°C in 5% CO₂. Dendritic cells were dislodged from the plastic surface by gently rapping the flask. Dendritic cells were then centrifuged at 600 xg for 5 minutes, washed in DPBS and counted as described above.
(4) The prepared dendritic cells were placed into a 96 well round bottom plate at a concentration of 2x10⁴ cells/well in 100 microliter total volume of AIM V media, per well.
   CD4+ T cells were prepared from frozen aliquots of the peripheral blood cell samples used to prepare the dendritic cells, using reagents provided by the Dynal CD4+ T-cell enrichment kit (Dynal). The resultant CD4+ cell solution was centrifuged, resuspended in AIMV media and the cell density was determined using methods known in the art. The CD4+ T-cell suspension was then resuspended to a count of 2x10⁶/ml in AIM V media to facilitate efficient manipulation of 96-well plates.

### EXAMPLE 2

### Identification of T-Cell Epitopes in IFN-β

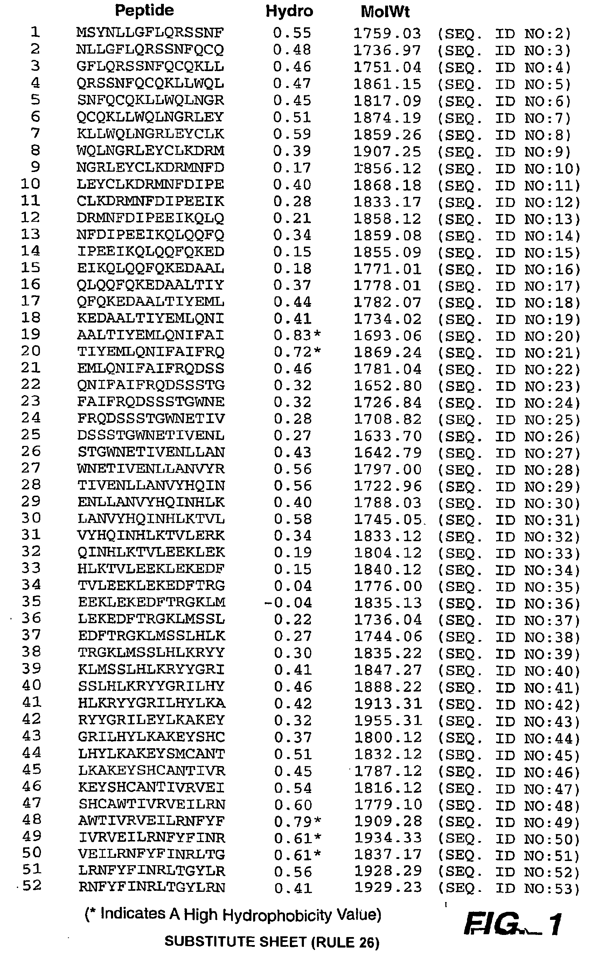

Peptides for use in the I-MUNE® assay described in Example 3 were prepared based on the sequence of human fibroblast IFN-β sequence (Genbank P01574) with the sequence: 
msynllgflqrssnfqcqkllwqlngrleyclkdrmnfdipeeikqlqqfqkedaaltiyemlqnifaifrqdssstgwnetivenlla
nvyhqinhlktvleeklekedftrgklmsslhlkryygrilhylkakeyshcawtivrve ilrnfyfinrltgylrn (SEQ ID
NO:1)
 Based upon the full length amino acid sequence (SEQ ID NO:1) of this IFN-β, 15mers comprising the entire sequence of IFN-β were synthetically prepared. Consecutive peptides overlapped by 12 amino acids. A total of 52 peptides (SEQ ID NOS: 2-53) were created, the sequences of which are provided in Figure 1.
Peptide antigen was prepared as a 2 mg/ml stock solution in DMSO. First, 0.5 microliters of the stock solution were placed in each well of the 96 well plate in which the differentiated dendritic cells were previously placed. Then, 100 microliters of the diluted CD4+ T-cell solution as prepared above, were added to each well. Useful controls include diluted DMSO blanks, and tetanus toxoid positive controls.
The final concentrations in each well, at 20 microliter total volume are as follows:
2x10⁴ CD4+
2x10⁵ dendritic cells (R:S of 10:1)
5 µM peptide

### EXAMPLE 3

### 1-MUNE® Assay for the Identification of Peptide T-Cell Epitopes in IFN-β Using Human T-Cells

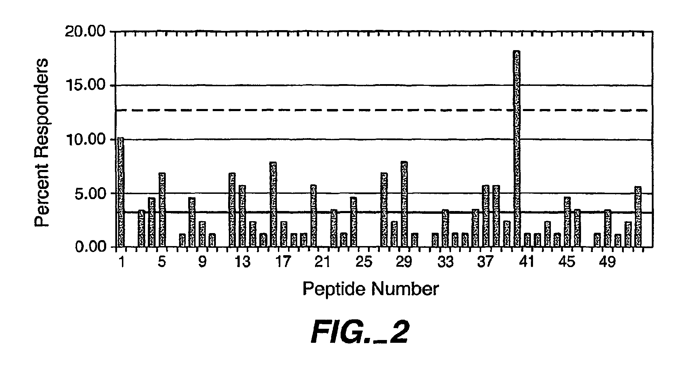

Once the assay reagents (*i.e*., cells, peptides, etc.) were prepared and distributed into the 96-well plates, the I-MUNE® assays were conducted. Controls included dendritic cells plus CD4+ T-cells alone (with DMSO carrier) and with tetanus toxoid (Wyeth-Ayerst), at approximately 5 Lf/mL.
Cultures were incubated at 37°C in 5% CO₂ for 5 days. Tritiated thymidine (NEN) was added at 0.5 microCl/well. The cultures were harvested and assessed for incorporation the next day, using the Wallac TriBeta scintillation detection system (Wallace Oy).
All tests were performed at least in duplicate. All tests reported displayed robust positive control responses to the antigen tetanus toxoid. Responses were averaged within each experiment, then normalized to the baseline response. A positive event (*i.e*., a proliferative response) was recorded if the response was at least 2.95 times the baseline response.
The immunogenic responses (*i.e*., T-cell proliferation) to the prepared peptides from human IFN-β were tallied and are shown in Figure 2. The overall background rate of responses to this peptide set was 3.17 ± 3.28%, for the donors tested. The percent response to peptide #40 was 18.18%, which was well above the background plus three standard deviations cut off level for significance of 13.01%. The percent response to peptide #1 reached a level of three-fold the background level (three-fold background = 9.51 %). These two peptides were selected for further analysis, along with peptide #27, which had been prominent prior to completion of the I-MUNE® assay experiments.

### EXAMPLE 4

### I-MUNE® Assay of Variant Peptides

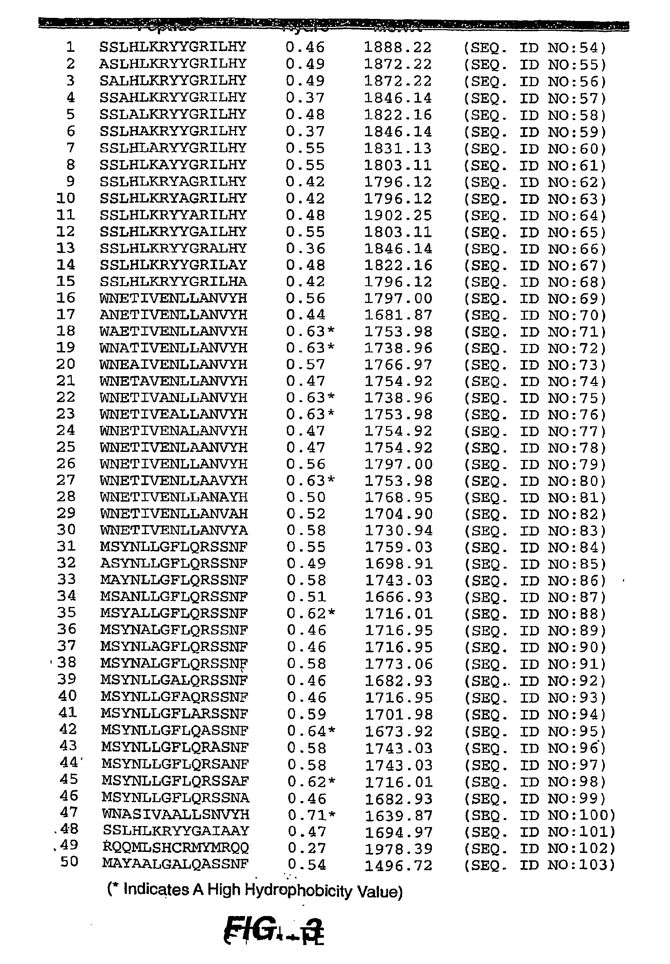

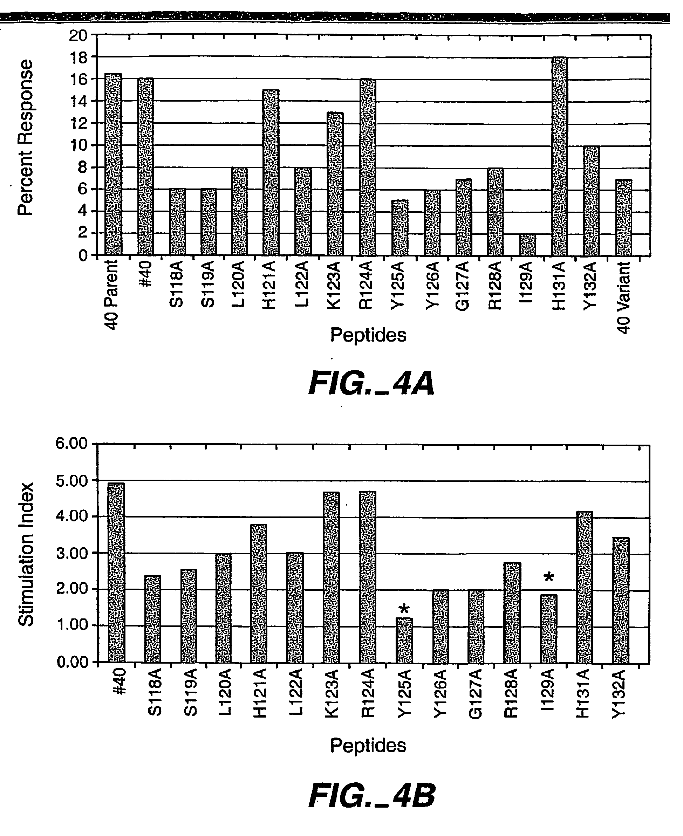

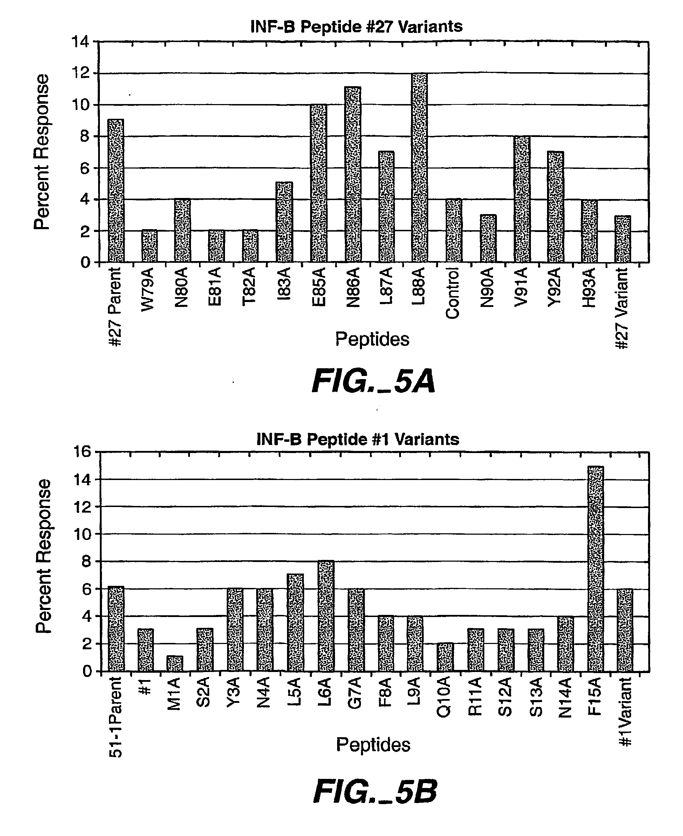

A set of variant peptides was ordered based on the sequences of peptides #1 (SEQ ID NO:2), #27 (SEQ ID NO:28), and #40 (SEQ ID NO:41). The sequences of these peptides are shown in Figure 3. An alanine scan was performed for each peptide. In addition, variant peptides were ordered that corresponded to the peptide sequences as described by Runkel *et al*., (Runkel et al., Biochem., 39:2538-2551 [2000]). These variant peptides were synthesized by Mimotopes (San Diego, CA) using the multi-pin synthesis technique known in the art (*See e.g*., Maeji et al., J. Immunol. Meth., 134:23-33 [1990]).
The I-IMUNE® assay was performed as described in Example 3, utilizing the variant peptides on a set of 103 donor samples. Proliferative responses were collated, and the result is shown in Figure 4. Additional testing of peptides #1 and #27 confirmed their non-statistically relevant status (*See*, Figure 5). Epitope #40 returned a consistent donor response percent of 15%. The peptide variant I129A induced the lowest level of proliferative responses in this donor set (Figure 4, Panel A). Y125A was also effective at reducing responses. I129A and Y125A induced statistically significant reduction in overall proliferation as compared to the parent peptide sequence when responders to the parent peptide were analyzed in a two-tailed parametric Students' t-test (P≤ 0.001 for both comparisons; *See*, Figure 4, Panel B).
Based on the known crystal structure of human IFN-β, a number of alternative sequences in the peptide #40 region were selected that were predicted to have little effect on the protein structure, but that were different enough to potentially affect the recognition of the epitope by the immune system. The variants selected for further characterization are listed in Table 1.

**Table 1. IFN-β Variants**

| | | | | | | |
|---|---|---|---|---|---|---|
| 1 | C17S | | | | | |
| 2 | C17S | S118D | | | | |
| 3 | C17S | Y125A | | | | |
| 4 | C17S | Y125L | | | | |
| 5 | C17S | Y126A | | | | |
| 6 | C17S | Y125K | Y126F | | | |
| 7 | C17S | I129L | | | | |
| 8 | C17S | I129A | | | | |
| 9 | C17S | I129V | | | | |
| 10 | C17S | R124S | | | | |
| 11 | C17S | R128Q | | | | |
| 12 | C17S | R124A | | | | |
| 13 | C17S | R128A | | | | |
| 14 | C17S | S2A | N4A | L5A | F8A | R11A |

### EXAMPLE 5

### HLA Association with Epitope Peptide #40

The HLA-DR and DQ expression of all the donors tested in both rounds of assay testing described above were assessed using a commercially available PCR-based HLA typing kit (Bio-Synthesis). The phenotypic frequencies of individual HLA-DR and -DQ antigens among responders and non-responders to peptide #40 were tested using a chi-squared analysis with 1 degree of freedom. The increased or decreased likelihood of reacting to epitope #40, the relative risk, was calculated wherever the HLA antigen in question was present in both responding and non-responding donor samples.
A total of 186 donors were completely HLA typed, and were included in the analysis for epitope peptide #40. The results of the statistical analysis are shown in Table 2.

**Table 2.**

| **Statistical Analysis of HLA Typing and Response to Epitope Peptide #40** | | | | |
|---|---|---|---|---|
| HLA antigen | chi-squared p | relative risk | percent responsive | percent non-responsive |
| HLA-DQ6 | p < 0.00005 | 3.58 | 61% | 25% |
| HLA-DR15 | p < 0.002 | 2.68 | 39% | 15% |
| HLA-DR1 | p < 0.05 | 0.19 | 17% | 3% |

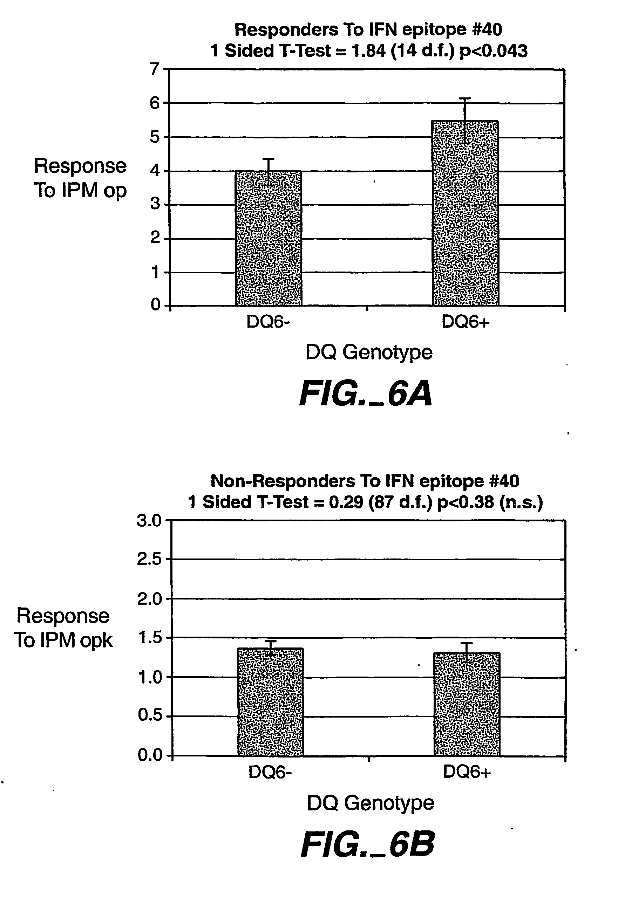

The presence of HLA-DQ6 is strongly correlated with response to epitope peptide #40. The presence of DR15 also associates with response to epitope peptide #40. However, the association between response to #40 and DQ6 is stronger than the linkage disequilibrium between DR15 and DQ6. These data indicate that the response is associated to DQ6 rather than to DR15.
The magnitude of the proliferative response to peptide #40 in responders and non-responders expressing HLA-DQ6 was also analyzed. The results are provided in Figure 6. A responder to the peptide is defined by a stimulation index of greater than 2.95: The proliferative response in donors who express HLA-DQ6 was found to be significantly higher than in peptide responders who do not express HLA-DQ6. As a control, non-responders to the peptide returned the same level of proliferation the peptide regardless of their expression of HLA-DQ6.
From the above, it is clear that the present invention provides methods and compositions for the identification of T-cell epitopes in the IFN-β protein sequence and the production of peptides which when incorporated into the IFN-β sequence, are no longer capable of initiating the CD4⁺T-cell response. In particular, the present invention provides means and compositions suitable for reducing the immunogenicity of IFN-β.

### EXAMPLE 6

### Identification of T-Cell Epitopes in Human Thrombopoietin

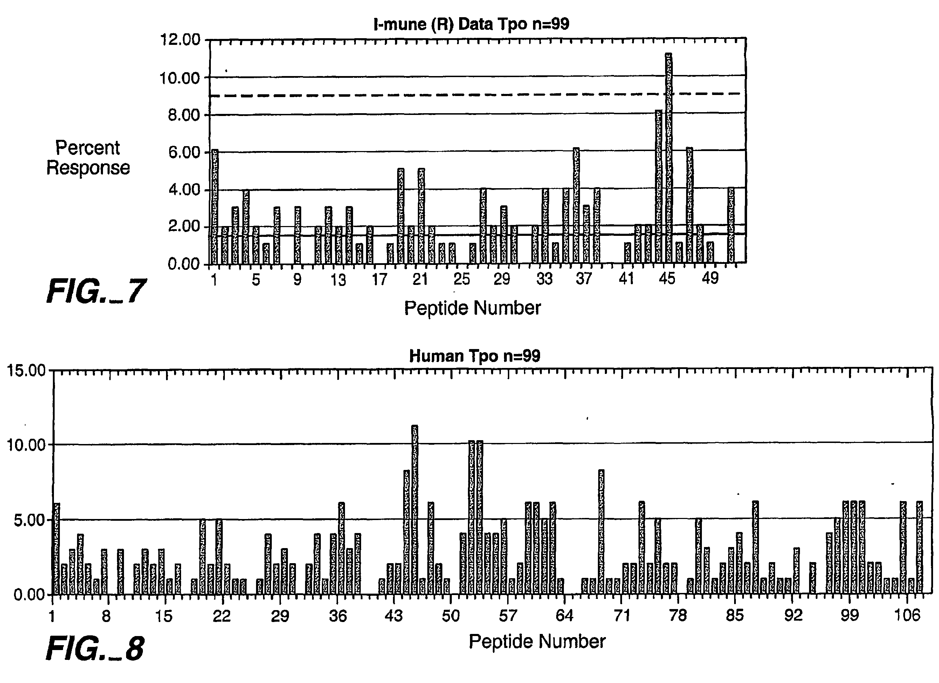

Peptides for use in the I-MUNE® assay as described in Example 3 were prepared based on the sequence of the first 152 residues of human thrombopoietin (*i.e*., the active domain of TPO), as well as the entire TPO sequence, including the CHO binding domain (GenBank Accession No. P40225) with the sequence: 
SPAPPACDLRVLSKLLRDSHVLHSRLSQCPEVHPLPTPVLLPAVDFSLGE
WKTQMEETKAQDILGAVTLLLEGVMAARGQLGPTCLSSLLGQLSGQVRLL
LGALQSLLGTQLPPQGRTTAHKDPNAIFLSFQHLLRGKVRFLMLVGGSTL
CVRRAPPTTAVPSRTSLVLTLNELPNRTSGLLETNFTASARTTGSGLLKW
QQGFRAKIPGLLNQTSRSLDQIPGYLNRIHELLNGTRGLFPGPSRRTLGA
PDISSGTSDTGSLPPNLQPGYSPSPTHPPTGQYTLFPLPPTLPTPVVQLH
PLLPDPSAPTPTPTSPLLNTSYTHSQNLSQEG (SEQ ID NO:104)
 The I-MUNE® assay described in Example 3, was conducted using cells obtained from 99 donors. The background was determined to be 2.5, and the standard deviation was 9.2. Figure 7 provides a graph showing the data obtained in the I-MUNE® assay with the first 152 residues of TPO (*i.e*., the active domain).
Figure 8 provides a graph showing the results for the I-MUNE® assay conducted using the entire TPO molecule, including the CHO binding domain. The epitope of interest (at peptide #45) was found to have the sequence RAPPTTAVPSRTSLVLTL (SEQ ID NO:105), and was found to have an association with HLA-DR14 of p=0.03.

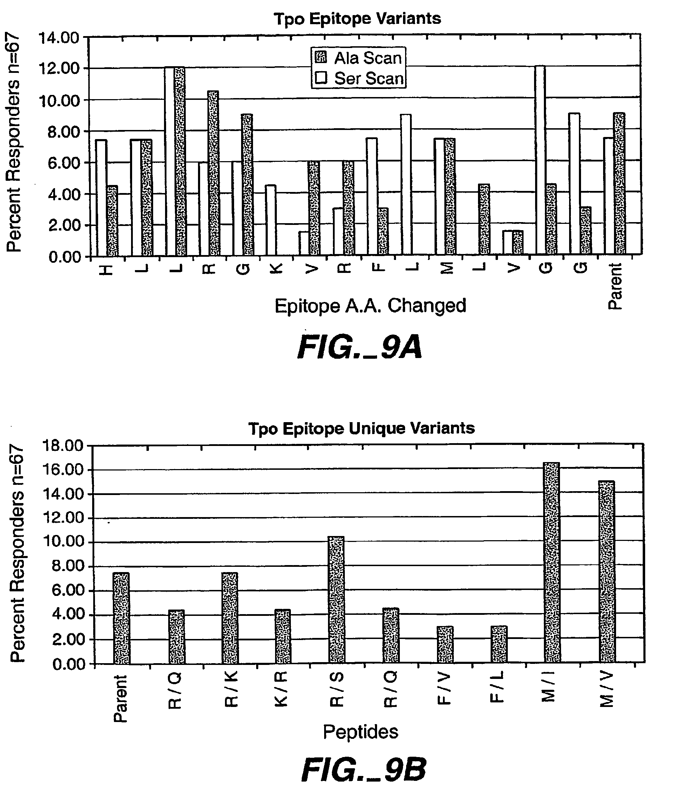

Figure 9, Panels A and B provide graphs showing the results for TPO epitope #45, in which the critical residues were tested. The average parental residue response was 9.18 ± 1.82. Values less that 3.5% were equivalent to 3 standard deviations under the averaged response of the parent peptides. These peptides were determined to be the preferred variants: K138S, V139A, R140A (this change has been described as an active variant; Park et al., J. Biol. Chem., 273:256-261 [1998]; and Hou and Zhan, Cytokine 10:319-330 [1998]), F141S, L142A, V143A, V143S, G145A, F141V, and F141L.
These data indicate the epitopes of interest and the results that were obtained using a selection of variant epitopes. It is contemplated that these variant epitopes will find use in various therapeutic protocols.

### EXAMPLE 7

### Identification of T-Cell Epitopes in Human SolubleTumor Necrosis Factor Receptor-1

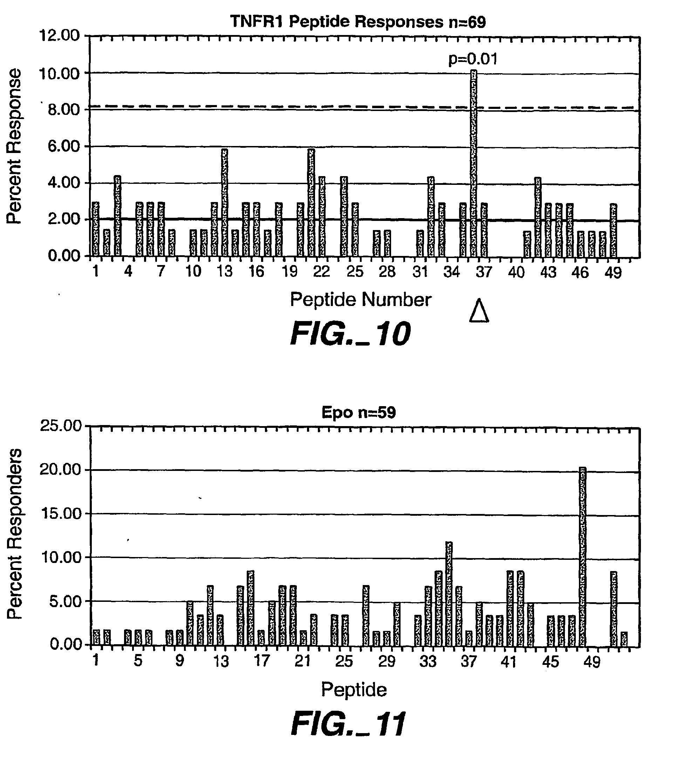

Peptides for use in the I-MUNE® assay as described in Example 3 were prepared based on the sequence of the soluble human tumor necrosis factor receptor 1 (TNF-R1) (GenBank Accession No. P19438) with the sequence: 
DSVCPQGKYI HPQNNSICCT KCHKGTYLYN DCPGPGQDTD CRECESGSFT
ASENHLRHCL SCSKCRKEMG QVEISSCTVD RDTVCGCRKN QYRHYWSENL
FQCFNCSLCL NGTVHLSCQE KQNTVCTCHA GFFLRENECV SCSNCKKSLE
CTKLCLPQIE N (SEQ ID NO:106)
 The background was determined to be 2.23 and the value for three standard deviations was 8.07. One epitope of interest was identified, as shown in Figure 10. This epitope has the sequence CSLCLNGTVHLSCQE (SEQ ID NO:107). This significant epitope was found to be in a region of the protein that was truncated when a variant molecule was created. This variant was then reported to have a reduced immunogenic capacity when tested in a non-human primate (*See*, Rosenberg et al., J. Appl. Physiol., 91:2213-2223 [2001]). Thus, it is contemplated that modifications in this epitope will provide advantages in the development of sTNFR-1 for use in treatment.

### EXAMPLE 8

### Identification of T-Cell Epitopes in Human Erythropoietin

Peptides for use in the I-MUNE® assay as described in Example 3 were prepared based on the sequence of human erythropoietin (EPO), (GenBank Accession No. P1588), with the sequence: 
MGVHECPAWL WLLLSLLSLP LGLPVLGAPP RLICDSRVLE RYLLEAKEAE
NITTGCAEHCSLNENITVPD TKVNFYAWKR MEVGQQAVEV WQGLALLSEA
VLRGQALLVN SSQPWEPLQLHVDKAVSGLR SLTTLLRALG AQKEAISPPD
AASAAPLRTI TADTFRKLFR VYSNFLRGKLKLYTGEACRT GDR (SEQ ID NO:108)
 The I-MUNE® assay described in Example 3, was conducted using cells obtained from 59 donors. Figure 11 provides a graph showing the data obtained in the I-MUNE® assay with the pepset produced from the EPO sequence above (SEQ ID NO:1108). Epitopes of interest were identified as peptide #34 and peptide #48. Peptide #34 has the sequence SGLRSLTTLLRALGA (SEQ ID NO: 109), and peptide #48 has the sequence FRVYSNFLRGKLKLY (SEQ ID NO:110). As active mutants have been made in the region represented by peptides #34 and #48 (*See*, Elliott et al., 89:493-502 [1997]), it is contemplated that amino acid modifications in or around these peptides will provide variant EPO suitable for use as an EPO substitute.

## Claims

1. Variant protein having at least 80% sequence identity with SEQ ID No: 1, wherein said variant protein exhibits an altered immunogenic response as compared to interferon-beta as set out in SEQ ID No: 1, and comprising a CD4+ epitope as set forth in SEQ ID No: 41 in which CD4+ epitope at least one amino acid is substituted at an amino acid residue position selected from the group consisting of position 125 (Y) and position 129 (I).

2. Variant protein according to claim 1, wherein said substitution at position 125 is selected from the group consisting of alanine (Y125A), leucine (Y125L), and lysine (Y125K).

3. Variant protein of Claim 1, wherein said substitution at position 129 is selected from the group consisting of leucine (I129L), alanine (I129A), and valine (I129V).

4. Variant protein of Claim 1, wherein said variant protein comprises at least one amino acid substitution at an amino acid residue selected from the group consisting of position 2, position 4, position 5, position 8, position 11, position 17, position 118, position 124, and position 128.

5. A composition comprising a nucleic acid encoding a variant protein according to any of the claims 1 to 4.

6. An expression vector encoding a variant protein according to any of the claims 1 to 4.

7. A host cell transformed with the expression vector of claim 6.

8. A composition comprising a variant protein according to any of the claims 1 to 4.

## Patentansprüche

1. Variantes Protein mit zumindest 80% Sequenzidentität mit SEQ ID Nr. 1, wobei das variante Protein im Vergleich zu dem Beta-Interferon wie dargestellt in SEQ ID Nr. 1 eine veränderte Immunogen-Antwort zeigt, und ein CD 4+ Epitop wie dargelegt in SEQ ID Nr. 41 aufweist, wobei in dem CD 4+ Epitop zumindest eine Aminosäure an zumindest einer Aminosäurerest-Position, ausgewählt aus der Gruppe, die aus Position 125 (Y) und Position 129 (I) besteht, substituiert ist.

2. Variantes Protein nach Anspruch 1, wobei die Substitution an Position 125 ausgewählt ist aus der Gruppe, die aus Alanin (Y125A), Leucin (Y125L), und Lysin (Y125 K) besteht.

3. Variantes Protein nach Anspruch 1, wobei die Substitution an Position 129 ausgewählt ist aus der Gruppe, die aus Leucin (I129L), Alanin (I129A) und Valin (I129V) besteht.

4. Variantes Protein nach Anspruch 1, wobei das variante Protein zumindest eine Aminosäurensubstitution an zumindest einem Aminosäurerest, ausgewählt aus der Gruppe, die aus Position 2, Position 4, Position 5, Position 8, Position 11, Position 17, Position 118, Position 124 und Position 128 besteht, aufweist.

5. Zusammensetzung aufweisend eine Nukleinsäure, die ein variantes Protein nach irgendeinem der Ansprüche 1 bis 4 kodiert.

6. Expressionsvektor, der ein variantes Protein gemäß irgendeinem der Ansprüche 1 bis 4 kodiert.

7. Wirtszelle, die mit dem Expressionsvektor nach Anspruch 6 transformiert wurde.

8. Zusammensetzung, aufweisend ein variantes Protein nach irgendeinem der Ansprüche 1 bis 4.

## Revendications

1. Protéine variante ayant au moins 80 % d'identité de séquence avec SEQ ID NO: 1, où ladite protéine variante exhibe une réponse immunogène altérée par comparaison à l'interféron bêta tel que décrit dans SEQ ID NO: 1, et comprenant un épitope CD4+ tel que décrit dans SEQ ID NO: 41, épitope CD4+ dans lequel au moins un acide aminé est substitué à une position de résidu d'acide aminé sélectionnée dans le groupe consistant en la position 125 (Y) et la position 129 (I).

2. Protéine variante selon la revendication 1, dans laquelle ladite substitution à la position 125 est sélectionnée dans le groupe consistant en une alanine (Y125A), une leucine (Y125L), et une lysine (Y125K).

3. Protéine variante selon la revendication 1, dans laquelle ladite substitution à la position 129 est sélectionnée dans le groupe consistant en une leucine (I129L), une alanine (I129A), et une valine (I129V).

4. Protéine variante selon la revendication 1, où ladite protéine variante comprend au moins une substitution d'acide aminé à un résidu d'acide aminé sélectionné dans le groupe consistant en la position 2, la position 4, la position 5, la position 8, la position 11, la position 17, la position 118, la position 124, et la position 128.

5. Composition comprenant un acide nucléique codant pour une protéine variante selon l'une quelconque des revendications 1 à 4.

6. Vecteur d'expression codant pour une protéine variante selon l'une quelconque des revendications 1 à 4.

7. Cellule hôte transformée avec le vecteur d'expression selon la revendication 6.

8. Composition comprenant une protéine variante selon l'une quelconque des revendications 1 à 4.
